# EUROPEAN PATENT APPLICATION

(11) **EP 4 768 009 A2**
(43) Date of publication of application: **01.07.2026**
(21) Application number: 25213264.2
(22) Date of filing: 25.05.2022
(51) Int. Cl.: A61K 8/02

(54) **COMPOSITIONS AND METHODS FOR CONTROLLING SWEAT PRODUCTION**

(30) Priority: 07.06.2021 EP 21178027
(62) Divisional of application: 22730803.8
(71) Applicant: Unilever IP Holdings B.V., 6708 WH Wageningen (NL); Unilever Global IP Limited, Wirral, Merseyside CH62 4ZD (GB)
(72) Inventor: EVANS, Richard, Livesey, 6708 WH Wageningen (NL); WATERFIELD, Philip, Christopher, 6708 WH Wageningen (NL)
(74) Representative: Unilever Patent Group

(57) **Abstract**

An antiperspirant composition comprising a non-aluminium, non-zirconium, gel-forming, film-forming or water absorbing agent and an agent that affects the secretory coil of eccrine glands in such a manner as to reduce secretions therefrom.

## Description

### Field of Invention

The present invention is in the field of antiperspirancy, in particular methods of reducing perspiration without the use of aluminium and/or zirconium salts.

### Background of the Invention

There have been several publications published relating to the reduction of sweating using actives that do not block the sweat glands or pores.

WO 02/011690 (Unilever) discloses an antiperspirancy method whereby calcium channels within the secretory coil cells of the eccrine glands are blocked, thereby controlling sweat production at its source.

US 8,618,160 B2 (Rose U) discloses wipes containing glycopyrrolate, a muscarinic anticholinergic, as a treatment for hyperhidrosis (excessive perspiration).

Certain anticholinergic drugs have also been used to treat hyperhidrosis, with varying degrees of success. These drugs include Ditropan^{®} (oxybutynin), Probanthine^{®} (propantheline bromide) and Cogentin^{®} (benzotropine).

US 2008/0207737 (Zinger) discloses a topical composition for antiperspirant benefit comprising varying levels of oxybutynin.

### Summary of the Invention

It is an object of the invention to provide antiperspirant compositions that do not require pore-blocking aluminium or zirconium salts, such as those astringent salts used in traditional antiperspirant compositions.

It is a further object of the invention to provide antiperspirant compositions that have a dual mode of action, thereby providing enhanced wetness control on the skin of the human body.

It is a further object of the invention to provide compositions for reducing perspiration with at least reduced blockage of sweat glands compared with traditional antiperspirant compositions.

In a first aspect of the invention, there is provided an antiperspirant composition comprising; a non-aluminium, non-zirconium, gel-forming, film-forming or water absorbing agent; an agent that affects the secretory coil of eccrine glands in such a manner as to reduce secretions therefrom, termed a sweat secretory coil affecter, and a cosmetically acceptable carrier.

In a second aspect of the invention, there is provided a cosmetic method of reducing perspiration comprising the topical application of an antiperspirant composition according to the first aspect of the invention.

The second aspect of the invention described immediately above may alternatively be expressed as the use of an antiperspirant composition according to the first aspect of the invention for reducing perspiration on the surface of the human body,

In a third aspect of the invention, there is provided a method manufacture of an antiperspirant composition according to the first aspect of invention, said method comprising the bringing together of a non-aluminium, non-zirconium, gel-forming, film-forming or water absorbing agent and an agent that affects the secretory coil of eccrine glands in such a manner as to reduce secretions therefrom, termed a sweat secretory coil affecter, and a cosmetically acceptable carrier.

In a fourth aspect of the invention, there is provided a composition according to the first aspect of the invention for the topical treatment of hyperhidrosis.

**In** a fifth aspect of the invention, there is provided an antiperspirant composition according to the first aspect of the invention and a dispenser for said composition, the dispenser comprising containment means and application means for the composition.

### Detailed Description of the Invention

Herein, cosmetic methods and uses should be understood to be non-therapeutic methods, unless otherwise stated.

Herein, excessive perspiration should be understood to refer to that condition otherwise known as hyperhidrosis.

Herein, all percentages, parts, and ratios are by weight, unless otherwise indicated.

Herein, references to an amount of a compound or an active refer to the total amount of compounds or actives of the type indicated.

Herein, "application" and "applied" relate to application to the surface of the human body, in particular the underarm regions, unless the context dictates otherwise.

Herein, features expressed as "preferred" with regard to a particular aspect of the invention should be understood to be preferred with regard to each aspect of the invention (likewise, features expressed as "particularly preferred" or "especially preferred").

Herein, preferred, particularly preferred and especially preferred features of the invention are particularly preferred when used in combination with other preferred, particularly preferred and especially preferred features of the invention. This is particularly the case with combinations of a non-aluminium, non-zirconium, gel-forming agent and the sweat secretory coil affecter.

Herein, "ambient conditions" refer to 20°C and 1 atmosphere pressure, unless otherwise indicated.

Use of the invention typically involves the topical application of the antiperspirant composition to the underarm regions of the human body, otherwise known as the axillae.

An essential component of the invention is a non-aluminium, non-zirconium, gel-forming, film-forming or water absorbing agent. Such agents have an effect on the skin of the human body when applied thereto and typically reduce the amount of perspiration thereon. Preferred components of this type are gel-forming agents.

Herein, non-aluminium, non-zirconium gel-forming agents absorb water and form a gel-like structure on application to the surface of the human body.

The non-aluminium, non-zirconium, gel-forming, film-forming or water absorbing agent may be present in the composition at a level of from 0.5 to 30%, preferably from 1 to 25% and more preferably from 2 to 20%, these figures excluding any propellant that may also be present in the composition.

Preferred non-aluminium, non-zirconium gel-forming agents are selected from a list consisting of: (i) an amphiphilic material that forms a water-insoluble, liquid crystal phase of greater than one dimensional periodicity upon contact with perspiration; (ii) a lamellar phase stabilised oil-in-water emulsion; (iii) a C18-C22 fatty acid soap; (iv) a mixture of a C10-C22 liquid fatty acid and a water-soluble calcium or zinc salt capable of forming a water-insoluble salt with the C10-C22 fatty acid at a pH of greater than 6; (v) an oil-in-water emulsion comprising lipophilic material having a melting point greater than 40°C and (vi) a complex of a zinc salt with a β-amino alcohol.

Suitable amphiphilic materials that forms a water-insoluble, liquid crystal phase of greater than one dimensional periodicity upon contact with perspiration are described in EP 550,960 A1 (Unilever, 1992) and WO 94/024993 (Unilever, 1994). A preferred amphiphilic material of this type is a mixture consisting of isostearyl alcohol and glycerol monolaurate at a ratio of from 25: 75 to 45: 55.

Suitable lamellar phase stabilised oil-in-water emulsions and their use as antiperspirants are described in EP 2442779 B1 (Unilever, 2013). Preferred lamellar phase stabilised oil-in-water emulsions comprise an oil selected from hydrocarbon oils and ester oils, particularly triglyceride oils, such as sunflower seed oil. Other particular oils that might be used are C₁₂₋₁₅ alkyl benzoate esters, hydrogenated polybutene, PPG-14 butyl ether, triethyl citrate, isopropyl palmitate, and isopropyl myristate. The lamellar phase is preferably comprised of two non-ionic surfactants, one having a relatively low HLB (less than 8 and preferably less than 5) and one having a relatively high HLB (more than 12 and preferably more than 15). The ratio high HLB surfactant to low HLB surfactant is chosen so as to give a stable dispersion, preferred ratios being from 1:1 to 1:6, respectively. A blend of steareth-2 and steareth-20 has been found to be particularly preferred, especially when used in combination with sunflower seed oil. Steareth-2 and steareth-20 are most preferably used at a ratio of from 1:4 to 1:5.

Suitable C18-C22 fatty acid soaps and their use as antiperspirants are described in WO2020/078931 A1. Sodium stearate is a preferred fatty acid soap of this type.

Suitable mixtures of a C10-C22 liquid fatty acid and a water-soluble calcium or zinc salt capable of forming a water-insoluble salt with the C10-C22 fatty acid at a pH of greater than 6 are described in WO2020/094568 A1 (Unilever, 2020). Preferred mixtures of this are emulsions. Zinc salts are particularly preferred and preferred liquid fatty acids are oleic and ricinoleic acid.

Suitable oil-in-water emulsions comprising lipophilic material having a melting point greater than 40°C are described in WO 2021/073988 (Unilever, 2021). Preferred lipophilic materials having a melting point greater than 40°C for incorporation in the oil-in-water emulsion are selected from hydrogenated oils, fatty alcohols, fatty esters, petroleum oil and wax. A particularly preferred lipophilic material is a hydrogenated oil selected from hydrogenated soybean oil, hydrogenated palm oil, hydrogenated corn oil, hydrogenated peanut oil, hydrogenated cottonseed oil, hydrogenated olive oil, hydrogenated avocado oil, hydrogenated castor oil, or any mixture thereof. Especially preferred hydrogenated oils are selected from hydrogenated soybean oil, hydrogenated palm oil, hydrogenated peanut oil, hydrogenated cottonseed oil, beeswax, tribehenin, or a mixture thereof.

Suitable complexes of a zinc salt with a β-amino alcohol are described in WO 2020/03414 A1 (Unilever, 2020). Preferred complexes of a zinc salt with a β-amino alcohol have a molar ratio of zinc salt to β-amino alcohol of from 1: 0.5 to 1: 3. Particularly preferred complexes of this type are selected from mono ethanol amine, di-ethanol amine, tri-ethanol amine or 2-amino-1-butanol. Suitable zinc salts for forming the complex are zinc chloride, formate, acetate, propionate, gluconate and citrate. These complexes are preferably used in anhydrous formulations.

In certain alternative embodiments, preferred non-aluminium, non-zirconium, gel-forming, film-forming or water absorbing agents are film-forming agents that are cellulosic film-forming polymers (FFPs). A particularly preferred agent of this type is a C1-C7 alkyl cellulose, preferably used in combination with an organic solvent and (i) a plasticizer selected from one or more of triethyl citrate, polypropylene oxide (with Tg lower than 25°C), glycerol monostearate, stearic acid, cetyl alcohol, triethyl phosphate, or mineral oil or (ii) a particulate filler which is MQ resin. A particularly preferred agent of this type is ethyl cellulose.

Particularly preferred gel-forming agents are selected from a list consisting of: (i) a mixture consisting of isostearyl alcohol and glycerol monolaurate at a ratio of from 25: 75 to 45: 55 by weight; (ii) a lamellar phase stabilised oil-in-water emulsion; (iii) a C18-C22 fatty acid soap; (iv) ) a mixture of oleic or ricinoleic acid and a water-soluble zinc salt; (v) an oil-in-water emulsion comprising lipophilic material having a melting point greater than 40°C and (vi) a complex of a zinc salt with a β-amino alcohol.

Especially preferred gel-forming agents are selected from a list consisting of: (i) a mixture consisting of isostearyl alcohol and glycerol monolaurate at a ratio of from 25: 75 to 45: 55 by weight; (ii) a lamellar phase stabilised oil-in-water emulsion and (iii) a C18-C22 fatty acid soap.

A further essential component of the invention is an agent that affects the secretory coil of eccrine glands in such a manner as to reduce secretions therefrom, termed a "secretory coil affecter" hereinafter.

The secretory coil affecter reduces sweat production at its source, the secretory coils of sweat glands themselves. It is understood that the secretory coil affecter permeates to the secretory coil of the sweat glands, said permeation generally being enhanced by other components with which the affecter is formulated.

Preferred secretory coil affecters are cosmetic actives, i.e. they are suitable for cosmetic use. Particularly preferred secretory coil affecters are natural or naturally derived.

The secretory coil affecter may be present in the composition at a level of from 0.1 to 20%, preferably from 0.2 to 10% and more preferably from 0.5 to 5%, these figures excluding any propellant that may also be present in the composition.

In preferred compositions, the secretory coil affecter is formulated with a skin penetration enhancer (SPE). Preferred SPEs are short chain polyhydric alcohols, in particular C2-C5 alcohols with 2-5 hydroxy groups, especially glycerol and propylene glycol. The SPE may be present at a level of from 0.5 to 90%, preferably from 1 to 50%, more preferably from 2 to 30% and most preferably from 3 to 12%, these figures excluding any propellant that may also be present in the composition.

Due to the differing modes of action of the non-aluminium, non-zirconium, gel-forming, film-forming or water absorbing agent and the sweat secretory coil affecter, a higher level of the former is generally required. Hence, the ratio of the non-aluminium, non-zirconium, gel-forming, film-forming or water absorbing agent to the sweat secretory coil affecter is preferably from 1: 1 to 1000: 1, more preferably from 5: 1 to 1000: 1 and especially from 10: 1 to 1000: 1.

Preferred secretory coil affecters are selected from a list consisting of (i) calcium channel blocking agents (CCBAs), particularly voltage-gated CCBAs; (ii) anticholinergic materials; (iii) store operated calcium entry (SOCE) protein affecters; (iv) electroneutral cation-chloride cotransporter (ECCC) inhibitors and (v) calcium-activated chloride channel (CACC) inhibitors.

Suitable CCBAs are described in US 6,632,422 B2 (Unilever, 2003) as having a molecular weight of less than 750, this being thought to enhance skin penetration.

Preferred CCBAs are voltage-gated CCBAs, particularly preferred are those selected from verapamil and methoxy-verapamil.

Safrole, tanshinone and magnolol may also be suitable for use as CCBAs.

Suitable anticholinergic materials and their use as antiperspirants are described in numerous publications. US 8,618,160 B2 (Rose U, 2013) discloses wipes containing glycopyrrolate, a muscarinic anticholinergic, as a treatment for hyperhidrosis. US 2008/0207737 (Zinger, 2008) discloses a topical composition for antiperspirant benefit comprising varying levels of oxybutynin, a further muscarinic anticholinergic.

Preferred anticholinergic materials are muscarinic anticholinergic materials.

Preferred anticholinergic materials are selected from oxybutynin, tolterodine, sulpiride, glycopyrrolate, benztropine and propanetheline.

Herein, SOCE protein affecters are materials that effect SOCE proteins, generally moderating their operation and thereby reducing Ca²⁺ entry into the eccrine cells and causing an antiperspirant effect. Particular SOCE protein affecters are described in EP 3,270,881 B1 (Unilever, 2018).

SOCE proteins of particular relevance are Orai 1, STIM 1, Orai 3, TRPC1 and STIM 2 proteins and affecters of these SOCE proteins are particularly preferred secretory coil affecters.

Preferred SOCE protein affecters are 6-gingerol, 6-gingerdiol, ethyl cinnamate, 1E,6E)-1,7-bis(3,4-dimethoxyphenyl)-4,4-dimethylhepta-1,6-diene-3,5-dione, 1,7-bis(4-hydroxy-3-methoxyphenyl) heptane-3,5-diol, 1,7-bis (3,4-dimethoxyphenyl)-4,4-dimethylheptane-3,5-diol, benzaldehyde, honokiol and capsaicin.

Preferred ECCC protein inhibitors are inhibitors of the sodium-potassium-2 chloride cotransporter 1 (NKCC1) in eccrine sweat glands. Such materials are described in DE102004052707 A (Beiersdorf, 2004).

Particularly preferred NKCC1 ECCC protein inhibitors are bumetanide, furosemide, azosemide and torasemide. An especially preferred NKCC1 ECCC is ARN23746: 3-(dimethylsulfamoyl)-4-(8,8,8-trifluorooctylamino)benzoic acid (see Savardi et al., Chem 6, 2073-2096, Aug. 06, 2020).

CACCs and inhibition thereof are described in WO2014/027050 (BRAIN AG, 2014).

Preferred CACC inhibitors are inhibitors of the TMEM16a channel (also known as anoctamin-1 [ANO-1]). Particularly preferred inhibitors of this sort are selected from the list di-gallic acid, tannic acid, T16Ainh-A01, Ani9, MONNA and Silibinin.

T16Ainh-A01 is 6-tert-butyl-2-(furan-2-carboxamido)-4,5,6,7-tetrahydrobenzo[b]-thiophene-3-carboxylic acid.

Ani9 is known as (4-chloro-2-methylphenoxy)-acetic acid [(2-methoxyphenyl)-methylene]hydrazide, 2-(4-chloro-2-methylphenoxy)-N-[(2-methoxyphenyl)-methylideneamino]-acetamide, or 2-(4-chloro-2-methylphenoxy)-acetic acid 2-[(2-methoxyphenyl)methylene]hydrazide.

MONNA is N-((4-methoxy)-2-naphthyl)-5-nitroanthranilic acid.

Particularly preferred secretory coil affecters are selected from a list consisting of (i) inhibitors of Orai 1, STIM 1, Orai 3, TRPC1 or STIM 2 proteins and (ii) muscarinic anticholinergics.

Especially preferred secretory coil affecters are selected from a list consisting of glycopyrrolate, oxybutynin, propantheline bromide, benzotropine, 6-gingerol, 6-gingerdiol, ethyl cinnamate, 1E,6E)-1,7-bis(3,4-dimethoxyphenyl)-4,4-dimethylhepta-1,6-diene-3,5-dione, 1,7-bis(4-hydroxy-3-methoxyphenyl) heptane-3,5-diol, 1,7-bis (3,4-dimethoxyphenyl)-4,4-dimethylheptane-3,5-diol, benzaldehyde, honokiol and capsaicin.

The carrier used in conjunction with the present invention should be cosmetically acceptable and aid in the delivery of the antiperspirants agent to the surface of the human body. The antiperspirant agents are typically suspended or dissolved in the carrier. The carrier is typically a fluid, i.e. a liquid or gas at ambient temperature and pressure.

The carrier preferably comprises from 10 to 99% of the composition.

A preferred additional component of the carrier is ethanol. This may comprise up to 80% of the total composition, but is typically restricted to less than 70%, and often less than 60%. When employed ethanol typically comprises at least 10%, preferably at least 20%, and more preferably at least 40% by weight of total composition. Each of these preferred minimum levels of ethanol may be limited by the aforementioned maximum levels of ethanol. In some compositions, ethanol may serve as a skin penetration enhancer for the secretory coil affecter.

A preferred additional component of the carrier is water, especially when employed in conjunction with ethanol, to give an aqueous ethanol carrier. Water may comprise up to 90% of the total composition, but is typically restricted to less than 60%, and often less than 50%. When employed water typically comprises at least 10%, preferably at least 20%, and more preferably at least 30% by weight of total composition. Each of these preferred minimum levels of water may be limited by the aforementioned maximum levels of water.

A preferred additional component is a fragrance, typically at a level of from 0.1 to 5% of the total composition. In compositions also comprising water, the fragrance is preferably accompanied by a fragrance solubiliser, typically a non-ionic surfactant used a concentration of from 0.1 to 5% of the total composition.

A further optional component is an organic anti-microbial agent. Such agents may be selected from any of those known in the art, provided reasonable skill is used to avoid any incompatibilities. An organic anti-microbial agent is a particularly preferred additional component. When employed, organic anti-microbial agents are typically used at a level of from 0.1 to 5% by weight of the composition.

Thickening agents may be employed in compositions of the invention. Such agents increase the viscosity of or solidify the carrier in which the antiperspirant agent is typically suspended or dissolved.

Thickening agents may be selected from any of those known in the art, provided reasonable skill is used to avoid any incompatibilities. A preferred class of thickeners, especially for compositions also comprising water, are hydroxyalkyl celluloses, such as hydroxypropyl cellulose. When employed, thickening agents are typically used at a level of from 0.1 to 40%. When a hydroxyalkyl cellulose thickening agent is employed, this is typically used at a level of from 0.2 to 10% by weight.

Dispensers suitable for use with the present invention comprise containment means and application means for the composition. They also typically comprise means for getting the composition from its containment means to its application means.

Preferred dispensers are able to function without the consumer needing to touch the composition with his or her hands in order for it to be applied, this feature enhancing efficient delivery.

The composition of the invention can be applied cosmetically and topically to the skin by "contact" or non-contact" application". In contact methods, a composition is wiped across the surface of the skin, depositing a fraction of the composition as it passes. In non-contact methods, the composition is sprayed from a dispenser held proximate to the skin, onto the skin surface, the dispenser commonly being called an aerosol dispenser. Contact application generally involves (i) a liquid composition, which is usually applied using a roll-on dispenser or possibly absorbed into or onto a wipe, or (ii) a solid or soft solid composition typically comprising a carrier liquid that that is thickened by a structuring agent.

When the composition of the invention is delivered in a roll-on, a firm solid or a stick format, the topically acceptable carrier comprises a hydrophobic carrier or an aqueous carrier. The hydrophobic carrier in such cases may comprise a silicone compound, low boiling alcohol or a wax. When the composition comprises a propellant, it is delivered as an aerosol.

The composition of the present invention can comprise a wide range of other optional components. The CTFA Personal Care Ingredient Handbook, Second Edition, 1992, which is incorporated by reference herein in its entirety, describes a wide variety of nonlimiting personal care and pharmaceutical ingredients commonly used in the skin care industry, which are suitable for use in the compositions of the present invention. Examples include: antioxidants, binders, biological additives, buffering agents, colorants, thickeners, polymers, astringents, conditioners, exfoliating agents, pH adjusters, preservatives, natural extracts, essential oils, skin sensates, skin soothing agents, and skin healing agents.

### Examples

Compositions according to the invention are illustrated in Table 1 to 3. These may be prepared by method known in the art.

**Table 1**

| **Component** | **Example** (% by wt.) | | | | | | |
|---|---|---|---|---|---|---|---|
| | **1** | **2** | **3** | **4** | **5** | **6** | **7** |
| 6-gingerol | 0.5 | -- | -- | -- | -- | -- | 0.5 |
| Oxybutynin | -- | 0.5 | -- | -- | -- | -- | -- |
| Benzaldehyde | -- | -- | 0.8 | -- | -- | -- | -- |
| 6-gingerdiol | -- | -- | -- | 1.0 | -- | -- | -- |
| Honokiol | -- | -- | -- | -- | 1.0 | -- | -- |
| Capsaicin | -- | -- | -- | -- | -- | 0.5 | -- |
| Hydroxypropyl Cellulose | -- | -- | 1.7 | 1.5 | -- | 1.0 | 0.5 |
| Propylene glycol | 1.0 | 10.0 | 8.0 | -- | 2.00 | 8.0 | 5.0 |
| Glycerol | 1.0 | 3.0 | -- | -- | 1.2 | -- | 0.8 |
| Isostearyl alcohol | 4.5 | -- | -- | -- | 4.5 | -- | -- |
| Glycerol monolaurate | 6.75 | -- | -- | -- | 6.75 | -- | -- |
| Sodium stearate | -- | 10.0 | -- | -- | -- | -- | -- |
| Zinc-2A1B* | -- | -- | 12.0 | -- | -- | 10.0 | -- |
| Zinc chloride | -- | -- | -- | 1.7 | -- | -- | 2.0 |
| Oleic acid | -- | -- | -- | 7.0 | -- | -- | 7.5 |
| Cyclopentasiloxane | 40 | -- | -- | -- | 20 | -- | -- |
| Ethanol | To 100 | 60 | To 100 | 63 | To 100 | 45 | 55 |
| Water | -- | To 100 | -- | To 100 | -- | To 100 | To 100 |

| | | | | | | | |
|---|---|---|---|---|---|---|---|
| * Zinc complex of 2-amino-1-butanol. Molar ratio of zinc to 2S1B = 1: 2 | | | | | | | |

**Table 2**

| **Component** | **Example** (% by wt.) | | | | | | |
|---|---|---|---|---|---|---|---|
| | **8** | **9** | **10** | **11** | **12** | **13** | **14** |
| 6-gingerol | -- | 0.5 | -- | -- | -- | 0.7 | -- |
| Oxybutynin | 1.0 | -- | -- | -- | -- | -- | -- |
| Benzaldehyde | -- | -- | -- | 0.7 | -- | -- | -- |
| 6-gingerdiol | -- | -- | 1.0 | -- | -- | -- | -- |
| Honokiol | -- | -- | -- | -- | -- | -- | 0.5 |
| Capsaicin | -- | -- | -- | -- | 0.7 | -- | -- |
| Hydroxypropyl Cellulose | -- | -- | 1.7 | 1.5 | 1.0 | 1.0 | 0.5 |
| Propylene glycol | 1.0 | 10.0 | 8.0 | -- | 2.00 | 8.0 | 5.0 |
| Glycerol | 1.0 | 3.0 | -- | -- | 1.2 | -- | 0.8 |
| Isostearyl alcohol | 4.5 | -- | -- | -- | 4.5 | -- | -- |
| Glycerol monolaurate | 6.75 | -- | -- | -- | 6.75 | -- | -- |
| Sodium stearate | -- | 10.0 | -- | -- | -- | -- | -- |
| Zinc-2A1 B* | -- | -- | 12.0 | -- | -- | 10.0 | -- |
| Zinc chloride | -- | -- | -- | 1.7 | -- | -- | 2.0 |
| Oleic acid | -- | -- | -- | 7.0 | -- | -- | 7.5 |
| Cyclopentasiloxane | 20 | -- | -- | -- | 15 | -- | -- |
| Ethanol | To 100 | 60 | To 100 | 63 | To 100 | 45 | 55 |
| Water | -- | To 100 | -- | To 100 | -- | To 100 | To 100 |

| | | | | | | | |
|---|---|---|---|---|---|---|---|
| * Zinc complex of 2-amino-1-butanol. Molar ratio of zinc to 2S1B = 1: 2 | | | | | | | |

**Table 3**

| **Component** | **Example** (% by wt.) | | | | | | |
|---|---|---|---|---|---|---|---|
| | **15** | **16** | **17** | **18** | **19** | **20** | **21** |
| 6-gingerol | -- | -- | 1.0 | -- | 0.7 | -- | -- |
| Oxybutynin | -- | -- | -- | 0.5 | -- | -- | -- |
| Benzotropine | 0.8 | -- | -- | -- | -- | -- | -- |
| 6-gingerdiol | -- | 0.5 | -- | -- | -- | -- | -- |
| Honokiol | -- | -- | -- | -- | -- | 1.0 | -- |
| Capsaicin | -- | -- | -- | -- | -- | -- | 0.8 |
| Hydroxypropyl Cellulose | -- | -- | 1.7 | 1.5 | 1.0 | 1.0 | 0.5 |
| Propylene glycol | 1.0 | 10.0 | 8.0 | -- | 2.00 | 8.0 | 5.0 |
| Glycerol | 1.0 | 3.0 | -- | -- | 1.2 | -- | 0.8 |
| Isostearyl alcohol | 4.5 | -- | -- | -- | 4.5 | -- | -- |
| Glycerol monolaurate | 6.75 | -- | -- | -- | 6.75 | -- | -- |
| Sodium stearate | -- | 10.0 | - | -- | -- | -- | -- |
| Zinc-2A1 B* | -- | -- | 12.0 | -- | -- | 10.0 | -- |
| Zinc chloride | -- | -- | -- | 1.7 | -- | -- | 2.0 |
| Oleic acid | -- | -- | -- | 7.0 | -- | -- | 7.5 |
| Cyclopentasiloxane | 45 | -- | -- | -- | 40 | -- | -- |
| Ethanol | To 100 | 60 | To 100 | 63 | To 100 | 45 | 55 |
| Water | -- | To 100 | -- | To 100 | -- | To 100 | To 100 |

| | | | | | | | |
|---|---|---|---|---|---|---|---|
| * Zinc complex of 2-amino-1-butanol. Molar ratio of zinc to 2S1B = 1: 2 | | | | | | | |

## Claims

1. An antiperspirant composition comprising a non-aluminium, non-zirconium, gel-forming, film-forming or water absorbing agent; an agent that affects the secretory coil of eccrine glands in such a manner as to reduce secretions therefrom, termed a sweat secretory coil affecter, and a cosmetically acceptable carrier.

2. An antiperspirant composition according to claim 1, that is free from aluminium or zirconium containing antiperspirant agents.

3. An antiperspirant composition according to claim 1 or claim 2, comprising a non-aluminium, non-zirconium, gel-forming antiperspirant agent.

4. An antiperspirant composition according to any one of preceding claims, wherein the secretory coil affecter is selected from list A consisting of: (i) calcium channel blocking agents (CCBAs), particularly voltage-gated CCBAs; (ii) anticholinergic materials; (iii) store operated calcium entry (SOCE) protein affecters (iv) electroneutral cation-chloride cotransporter (ECCC) inhibitors, and (v) calcium-activated chloride channel inhibitors.

5. An antiperspirant composition according to claim 3 or claim 4, wherein the non-aluminium, non-zirconium gel-forming antiperspirant agent is selected from list B, consisting of: (i) an amphiphilic material that forms a water-insoluble, liquid crystal phase of greater than one dimensional periodicity upon contact with perspiration; (ii) a lamellar phase stabilised oil-in-water emulsion; (iii) a C18-C22 fatty acid soap; (iv) a mixture of a C10-C22 liquid fatty acid and a water-soluble calcium or zinc salt capable of forming a water-insoluble salt with the C10-C22 fatty acid at a pH of greater than 6; (v) an oil-in-water emulsion comprising a lipophilic material having a melting point greater than 40°C and (vi) a complex of a zinc salt with a β-amino alcohol.

6. An antiperspirant composition according to any one of the preceding claims, wherein the secretory coil affecter is selected from list A(1) consisting of (i) inhibitors of Orai 1, STIM1, Orai 3, TRPC1 or STIM2 proteins and (ii) muscarinic anticholinergics.

7. An antiperspirant composition according to any one of the preceding claims, wherein the secretion coil affecter is selected from list A(2) consisting of glycopyrrolate, oxybutynin, propantheline bromide; benzotropine, 6-gingerol, 6-gingerdiol, ethyl cinnamate, 1E,6E)-1,7-bis(3,4-dimethoxyphenyl)-4,4-dimethylhepta-1,6-diene-3,5-dione, 1,7-bis(4-hydroxy-3-methoxyphenyl) heptane-3,5-diol, 1,7-bis (3,4-dimethoxyphenyl)-4,4-dimethylheptane-3,5-diol, benzaldehyde, honokiol and capsaicin.

8. An antiperspirant composition according to claim any one of claims 4 to 7, wherein the non-aluminium, non-zirconium gel-forming antiperspirant agent is selected from list B(1), consisting of (i) a mixture consisting of isostearyl alcohol and glycerol monolaurate isostearyl alcohol and glycerol monolaurate at a ratio of from 25: 75 to 45: 55 by weight; (ii) a lamellar phase stabilised oil-in-water emulsion; (iii) a C18-C22 fatty acid soap; (iv) a mixture of oleic or ricinoleic acid and a water-soluble zinc salt; (v) an oil-in-water emulsion comprising lipophilic material selected from hydrogenated soybean oil, hydrogenated palm oil, hydrogenated peanut oil, hydrogenated cottonseed oil, beeswax, tribehenin, or a mixture thereof; and (vi) a complex of a zinc salt with a β-amino alcohol at a molar ratio of from 1: 0.5 to 1: 3.

9. An antiperspirant composition according to claim 8, wherein the non-aluminium, non-zirconium gel-forming antiperspirant agent is selected from list B(2), consisting of (i) a mixture consisting of isostearyl alcohol and glycerol monolaurate at a ratio of from 25: 75 to 45: 55 by weight; (ii) a lamellar phase stabilised oil-in-water emulsion and (iii) a C18-C22 fatty acid soap.

10. An antiperspirant composition according to any one of the preceding claims, wherein non-aluminium, non-zirconium, gel-forming, film-forming or water absorbing agent is organic in nature.

11. An antiperspirant composition according to any one of the preceding claims, wherein the weight ratio of the non-aluminium, non-zirconium, gel-forming, film-forming or water absorbing agent to the sweat secretory coil affecter is from 1: 1 to 1000: 1.

12. An antiperspirant composition according to any one of the preceding claims, comprising a skin penetration enhancer.

13. An antiperspirant composition according to claims 12, wherein the skin penetration enhancer is a C2-C5 alcohols having from 2 to 5 hydroxy groups

14. A cosmetic method of reducing perspiration comprising the topical application of an antiperspirant composition according to any of the preceding claims.

15. A composition as disclosed in any of claims 1 to 13 for the topical treatment of hyperhidrosis.
